# EUROPÄISCHE PATENTSCHRIFT

(11) **EP 4 335 484 B1**
(45) Veröffentlichungstag und Bekanntmachung des Hinweises auf die Patenterteilung: **25.11.2026**
(21) Anmeldenummer: 23192624.7
(22) Anmeldetag: 22.08.2023
(51) Int. Cl.: A61M 39/12, B29C 45/14

(54) **BIOKOMPATIBLE UND/ODER LEBENSMITTELECHTE SCHLAUCH-BAUGRUPPE**
BIOCOMPATIBLE AND/OR FOOD-RESISTANT TUBE ASSEMBLY
ENSEMBLE TUBE BIOCOMPATIBLE ET/OU RESISTANT AUX ALIMENTS

(30) Priorität: 08.09.2022 DE 102022209380
(43) Veröffentlichungstag der Anmeldung: 13.03.2024
(73) Patentinhaber: Raumedic AG, 95213 Münchberg (DE)
(72) Erfinder: Prescher, Jörg, 97729 Ramsthal (DE)
(74) Vertreter: Schmitt-Nilson Schraud Waibel Wohlfrom Patentanwälte Partnerschaft mbB

(56) Entgegenhaltungen:
- US-A- 4 998 337
- US-A- 5 248 171
- US-B1- 6 432 345

## Beschreibung

Die Erfindung betrifft eine biokompatible und/oder lebensmittelechte Schlauch-Baugruppe zur Verbindung mindestens dreier Schlauch-Anschlüsse.

Eine derartige biokompatible und/oder lebensmittelechte Schlauch-Baugruppe ist, beispielsweise als T-Stück, X-Stück oder als Y-Stück, vom Markt her bekannt.

Bei der Fertigung der bekannten Schlauch-Baugruppe ergeben sich im Bereich einer Mündungsöffnung, in der ein Schlauchabschnitt der Schlauch-Baugruppe in einen weiteren Schlauchabschnitt der Schlauch-Baugruppe einmündet, Fertigungsprobleme insbesondere dann, wenn die Schlauch-Baugruppe durch Spritzgießen oder durch ein Pressverfahren hergestellt wird.

Die US 5 248 171 A beschreibt ein Schlauchsystem umfassend einen flexiblen Gummischlauch und einen starren, integralen und einheitlichen Kunststoffabzweig, der sich von einem ersten Abschnitt des flexiblen Gummischlauchs erstreckt, wobei dieser erste Abschnitt eine erste axial verlaufende Länge aufweist; der starre Kunststoffabzweig umfassend einen hülsenförmigen Teil, der um den ersten Abschnitt des flexiblen Gummischlauchs geformt ist und mit diesem ersten Abschnitt des flexiblen Gummischlauchs entlang eines äußeren Teils des ersten Abschnitts des flexiblen Gummischlauchs verbunden ist; der starre Kunststoffabzweig umfassend einen abzweigförmigen Teil, der integral und einheitlich mit dem hülsenförmigen Teil ist und sich von diesem hülsenförmigen Teil erstreckt; der abzweigförmige Teil erstreckt sich durch eine Öffnung im flexiblen Gummischlauch bis zum Hohlrauminneren des flexiblen Gummischlauchs; der flexible Gummischlauch weist einen zweiten Abschnitt auf, der sich im Wesentlichen über den ersten Abschnitt hinaus erstreckt, wobei der erste und der zweite Abschnitt den gleichen Innendurchmesser haben.

Die US 4 998 337 A beschreibt ein Verfahren zur Herstellung einer dauerhaften Verbindung zwischen mindestens zwei fertigen Metallrohren, umfassend: Bereitstellen eines Metallkörpers mit mindestens zwei Enden, Platzieren jedes der mindestens zwei fertigen Metallrohre an einem der mindestens zwei Enden des Metallkörpers, um mindestens zwei überlappende Körper-Rohr-Verbindungen zu bilden, lokales Eindrücken eines Teils der überlappenden Körper-Rohr-Verbindungen, um dort eine Öffnung zu erzeugen, Formen eines flüssigen Kunststoffmateria-Is auf den überlappenden Körper-Rohr-Verbindungen und in den Öffnungen, und Aushärten des flüssigen Kunststoffmaterials, um eine dauerhafte Verbindung zu erzeugen.

Die US 6,432,345 B1 offenbart ein Verfahren zur Herstellung einer Silikonschlauch-Baugruppe.

Es ist eine Aufgabe der vorliegenden Erfindung, eine biokompatible und/oder lebensmittelechte Schlauch-Baugruppe der eingangs erwähnten Art derart weiterzubilden, dass entsprechende Fertigungsprobleme vermieden sind.

Diese Aufgabe ist erfindungsgemäß gelöst durch eine medizinische Schlauch-Baugruppe mit den in Anspruch 1 angegebenen Merkmalen.

Erfindungsgemäß wurde erkannt, dass der die Mündungsöffnung begrenzende Mündungsdichtkörper Fertigungsprobleme in diesem Bereich vermeiden hilft. Der erste Schlauchabschnitt und der zweite Schlauchabschnitt der Schlauch-Baugruppe können aus Kunststoff gefertigt sein.

Die Schlauch-Baugruppe kann insbesondere den Anforderungen an die Biokompatibilität nach ISO 10993 entsprechen. Die Schlauch-Baugruppe kann zudem FDA(Food and Drug Administration)-Anforderungen entsprechen. Die Schlauch-Baugruppe kann der KTW-Leitlinie für Trinkwasserschläuche entsprechen. Die Schlauch-Baugruppe kann den Anforderungen an Lebensmittelbedarfsgegenstände gemäß der Bedarfsgegenständeverordnung genügen.

Die biokompatible und/oder lebensmittelechte Schlauch-Baugruppe kann Anwendung bei der Arzneimittelabgabe und/oder als medizinischer Konnektor beziehungsweise als Pharma-Konnektor finden. Die Schlauch-Baugruppe kann Teil einer Kathetereinrichtung sein. Die Schlauch-Baugruppe kann auch in der Lebensmittelindustrie zum Einsatz kommen.

Eine unerwünschte Gratbildung im Bereich der Mündungsöffnung kann durch Einsatz des Mündungsdichtkörpers vermieden werden. Auch eine Materialverformung, insbesondere eine Expansion der Schlauchabschnitte im Bereich der Mündungsöffnung kann vermieden werden. Soweit die medizinische Schlauch-Baugruppe durch Spritzgießen hergestellt wird, ergibt sich ein verbessertes Entformen der Schlauch-Baugruppe an einer Matrize, wobei insbesondere Formgebungsfehler (Moldingfehler), insbesondere eine Blasenbildung im Material der Schlauchabschnitte vermieden sind. Der Mündungsdichtkörper kann zudem für eine bessere Kraftverteilung in der Schlauch-Baugruppe im Bereich der Mündungsöffnung sorgen. Es ergibt sich eine erhöhte Stabilität der Schlauch-Baugruppe.

Die Ausführung des Mündungsdichtkörpers mit dem Hülsenabschnitt längs des einen Schlauchabschnitts und dem dem anderen Schlauchabschnitt teilumfänglich folgenden weiteren Dichtkörperabschnitt erleichtert die Fertigung der Schlauch-Baugruppe, insbesondere ein Entformen der gefertigten Schlauch-Baugruppe oder einer dem Mündungsdichtkörper aufweisenden Komponente von einem formgebenden Werkzeug.

Gestaltungen des Mündungsdichtkörpers nach den Ansprüchen 2 und 3 haben sich zur Gewährleistung eines unproblematischen Fertigungsverfahrens besonders bewährt.

Ausführungen nach Anspruch 4 haben sich in der Praxis bewährt. Bei der Ausführung "X-Stück" können zwei Mündungsdichtkörper zum Einsatz kommen, die jeweils eine der Mündungsöffnungen zu den Innenlumen der Schlauchabschnitte hin begrenzen.

Silikonmaterial nach Anspruch 1 haben sich für die medizinische/pharmazeutische Anwendung der Schlauch-Baugruppe bewährt.

Eine Shore-Härten-Relation nach Anspruch 5 führt zu einer verbesserten Stabilität der medizinischen Schlauch-Baugruppe im Bereich der Mündungsöffnung.

Ein Shore-Härte-Bereich nach Anspruch 6 hat sich in der Praxis bewährt. Die Shore-Härte des Mündungsdichtkörpers kann größer sein als 60 Shore-A und kann auch größer sein als 70 Shore-A.

Eine weitere Aufgabe der vorliegenden Erfindung ist es, ein Verfahren zum Spritzgießen oder Pressen einer biokompatiblen und/oder lebensmittelechten Schlauch-Baugruppe, was den Materialeinsatz angeht, effektiv eingesetzt werden kann.

Diese Aufgabe ist zumindest zunächst gelöst durch ein Verfahren mit dem in Anspruch 7 angegebenen Merkmalen.

Erfindungsgemäß wurde erkannt, dass es bei diesem Verfahren möglich ist, mittels der Matrize die Innenlumen der Schlauchabschnitte, abgesehen von einer direkten Umgebung der Mündungsöffnung, wo eine Innenlumen-Begrenzung durch den Mündungsdichtkörper stattfindet, vollständig durch die Matrize vorzugeben. Der Mündungsdichtkörper kann dann materialsparender ausgeführt sein, was zudem Vorteile bei den Zugmöglichkeiten für die Matrize bieten kann. Die Matrize kann insbesondere mit einem Formwerkzug, mit dem das Verfahren durchgeführt wird, mechanisch verbunden sein. Eine Aufwandsverringerung bei der Verfahrensdurchführung kann die Folge sein.

Die Vorteile eines Spritzgießverfahrens oder Pressverfahrens nach Anspruch 8 entsprechen denen, die vorstehend im Zusammenhang mit der medizinischen Schlauch-Baugruppe bereits erläutert wurden.

Die Matrize kann aus Metall gefertigt sein. Die Matrize kann mehrteilig sein. Der Mündungsdichtkörper und die Matrize stellen gemeinsam eine im Umspritzschritt umspritzte Komponentengruppe dar, wobei nach dem Umspritzen der Mündungsdichtkörper und die Schlauchabschnitte fest miteinander verbunden sind, sodass beim Ziehen der Matrize nur die Matrize, nicht aber der Mündungsdichtkörper gezogen wird.

Ein Ausführungsbeispiel der Erfindung wird nachfolgend anhand der Zeichnungen näher erläutert. In dieser zeigen:
- Fig. 1: einen Längsschnitt durch eine biokompatible und/oder lebensmittelechte Schlauch-Baugruppe zur Verbindung mindestens dreier Schlauch-Anschlüsse, ausgeführt als T-Stück;
- Fig. 2: die biokompatible und/oder lebensmittelechte Schlauch-Baugruppe nach Fig. 1 während eines Spritzgieß-Herstellungsverfahrens nach einem Umspritz-Schritt einer Matrize und vor einem Ziehen der Matrize; und
- Fig. 3: eine perspektivische Ansicht eines Abschnitts der biokompatiblen und/oder lebensmittelechten Schlauch-Baugruppe nach Fig. 1 im Bereich eines T-Anschlusses, wobei ein erster, gerade verlaufender Schlauchabschnitt in einem Längsschnitt mit im Vergleich zu den Figuren 1 und 2 senkrecht verlaufender Schnittachse gezeigt ist, sodass der Blick auf einen Mündungs-Dichtkörper der Schlauch-Baugruppe frei ist.

Eine biokompatible und/oder lebensmittelechte Schlauch-Baugruppe 1 dient zur Verbindung mindestens dreier Schlauch-Anschlüsse, die in der Zeichnung nicht näher dargestellt und durch die Kennzeichnungen "A", "B" und "C" symbolisiert sind. Die Schlauch-Baugruppe 1 kann im Klinikbereich, im medizinischen/pharmazeutischen Laborumfeld oder auch beim Abfüllen pharmazeutischer Fluide zum Einsatz kommen. Anwendung kann die Schlauch-Baugruppe 1 beispielsweise bei der Arzneimittelabgabe und/oder als medizinischer/Pharma-Konnektor finden. Eine weitere mögliche Anwendung der Schlauch-Baugruppe 1 ist in der Lebensmittelindustrie, beispielsweise beim Abfüllen flüssiger oder auch höherviskoser Lebensmittelmassen, wie z.B. pürierter/pastöser Massen.

Die Schlauch-Baugruppe 1 ist biokompatibel und lebensmittelecht.

Die Schlauch-Baugruppe 1 hat einen ersten Schlauchabschnitt 2 zur Verbindung der beiden Schlauch-Anschlüsse A und B. Ein zweiter Schlauchabschnitt 3 der Schlauch-Baugruppe 1 dient zur Verbindung mit dem dritten Schlauch-Anschluss C. Der zweite Schlauchabschnitt 3 mündet über eine Mündungsöffnung 4 in einer Mantelwand 5 des ersten Schlauchabschnitts 2 in den ersten Schlauchabschnitt 2 ein.

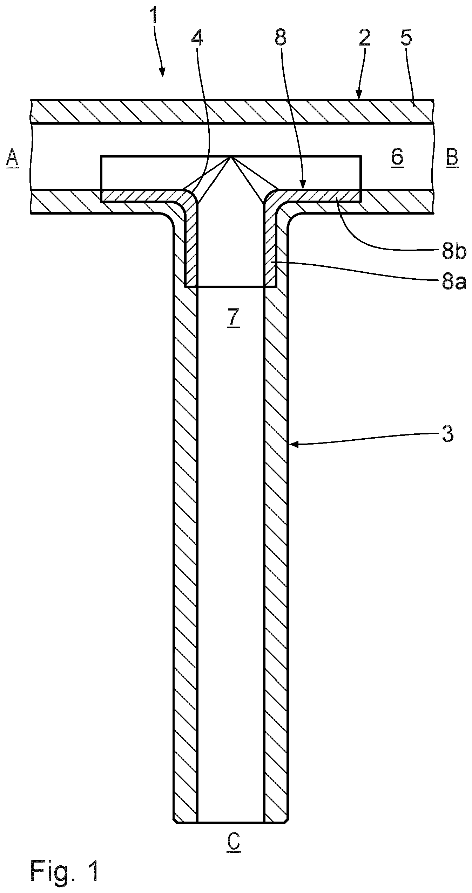

Im Bereich des ersten Schlauchabschnitts 2 ist die Schlauch-Baugruppe 1 nicht vollständig dargestellt. Der erste Schlauchabschnitt 2 kann in in der Fig. 1 horizontalen Richtung wesentlich weiter ausgedehnt sein.

Die beiden Schlauchabschnitte 2, 3 sind aus Kunststoff.

Die beiden Schlauchabschnitte 2, 3 sind aus Silikonmaterial gefertigt.

Die Mündungsöffnung 4 wird zu einem Innenlumen 6 des ersten Schlauchabschnitts 2 hin und zu einem Innenlumen 7 des zweiten Schlauchabschnitts 3 hin durch einen Mündungsdichtkörper 8 begrenzt.

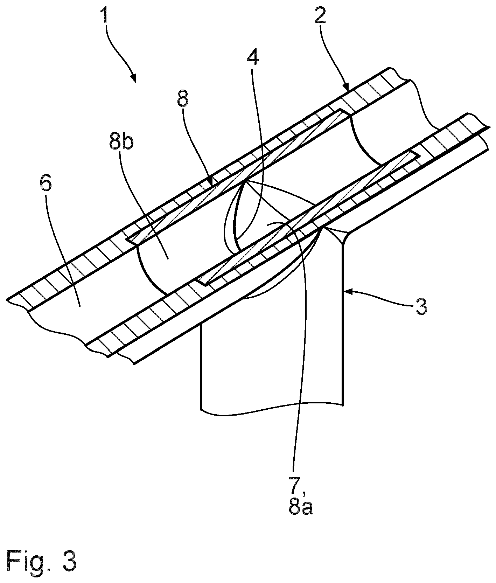

Die in den Figuren 1 bis 3 dargestellte Schlauch-Baugruppe 1 ist als T-Stück ausgeführt, wobei der erste Schlauchabschnitt 2 das obere Joch des Buchstabens "T" und der zweite Schlauchabschnitt 3 den hierzu senkrechten Schenkel des Buchstabens "T" bildet.

Der erste Schlauchabschnitt 2 und der zweite Schlauchabschnitt 3 sind an den Mündungsdichtkörper 8 angespritzt.

Der Mündungsdichtkörper 8 ist aus Silikonmaterial gefertigt. Eine Shore-Härte des Mündungsdichtkörpers 8 ist größer als eine Shore-Härte der Schlauchabschnitte 2, 3. Die Shore-Härte des Mündungsdichtkörpers 8 kann im Bereich zwischen 50 Shore-A und 80 Shore-A liegen.

Der Mündungsdichtkörper 8 hat einen Hülsenabschnitt 8a, der im Bereich der Mündungsöffnung 4 das Innenlumen 7 des zweiten Schlauchabschnitts 3 begrenzt. Weiterhin hat der Mündungsdichtkörper 8 einen im Grundsatz die Form eines Halbkreis-Zylindermantels aufweisenden weiteren Dichtkörperabschnitt 8b, der im Bereich der Mündungsöffnung 4 das Innenlumen 6 des ersten Schlauchabschnitts 2 über eine Hälfte des Innenlumen-Umfangs begrenzt.

Die beiden Abschnitte 8a, 8b des Mündungsdichtkörpers 8 sind einstückig aneinander angeformt.

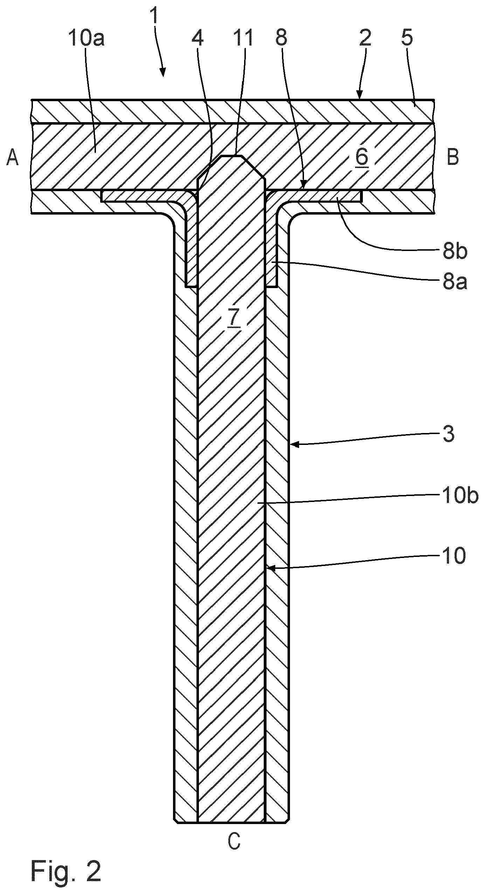

Beim Spritzgießen der medizinischen Schlauch-Baugruppe 1 wird folgendermaßen vorgegangen:
Zunächst wird eine Matrize 10 (vgl. Fig. 2) zur Vorgabe der Innenlumen 6, 7 der Schlauchabschnitte 2, 3 bereitgestellt. Die Matrize 10 kann aus Metall sein. Die Matrize 10 ist zweiteilig mit einem ersten Kernabschnitt 10a zur Vorgabe des Innenlumens 6 des ersten Schlauchabschnitts 2 und mit einem zweiten Kernabschnitt 10b zur Vorgabe des Innenlumens 7 des zweiten Schlauchabschnitts 3. Im Bereich der Mündungsöffnung 4 greifen die beiden Kernabschnitte 10a, 10b der Matrize 10 in einem Eingriffsbereich 11 ineinander ein. Beim Bereitstellen der Matrize 10 wird zunächst der Kernabschnitt 10a in ein entsprechendes, das Spritzgussvolumen vorgebendes Werkzeug eingelegt und anschließend der Kernabschnitt 10b in den Eingriffsabschnitt 11 eingeschoben.

Anschließend wird der Mündungsdichtkörper 8 auf den Kanalabschnitt 10b aufgeschoben, bis er über den weiteren Dichtkörperabschnitt 8b am ersten Kanalabschnitt 10a der Matrize 10 anliegt. Alternativ kann vor dem Einsetzen des zweiten Kernabschnitts 10b auch zunächst der Mündungsdichtkörper 8 auf dem ersten Kernabschnitt 10a der Matrize 10 so positioniert werden, dass der Hülsenabschnitt 8a mit dem Eingriffsbereich 11 der Matrize 10 fluchtet. Anschließend kann der zweite Kernabschnitt 10b durch den Hülsenabschnitt 8a des Mündungsdichtkörpers 8 in den Eingriffsbereich 11 eingeschoben werden, sodass die Matrize 10 vervollständigt ist.

Die somit insgesamt vervollständigte, zu umspritzende Spritzmatrize, umfassend die beiden Kernabschnitte 10a, 10b, also die Matrize 10, und den positionierten Mündungsdichtkörper 8, wird dann in die Spritzgussform des Spritzgusswerkzeugs eingelegt.

Anschließend werden die Matrize 10 und der Mündungsdichtkörper 8 mit fließfähigem Material der Schlauchabschnitte 2, 3, also mit dem Silikonmaterial der Schlauchabschnitte 2, 3, in der Spritzgussform umspritzt.

Nach erfolgtem Umspritzen und entsprechendem Aushärten des zunächst fließfähigen Materials der Schlauchabschnitte 2, 3 wird zunächst der Kernabschnitt 10b aus dem Lumen 7 des zweiten Schlauchabschnitts 3 und anschließend der Kernabschnitt 10a aus dem Lumen 6 des ersten Schlauchabschnitts 2 gezogen. Nach dem Entnehmen aus der Spritzgussform liegt dann die fertige Schlauch-Baugruppe 1 vor.

Nach dem Umspritzen sind der Mündungsdichtkörper 8 und die Schlauchabschnitte 2, 3 fest und unverlierbar miteinander verbunden.

Der Mündungsdichtkörper 4 sorgt dafür, dass im Bereich der Mündungsöffnung 4 keine unerwünschte Deformation der Wände der Schlauabschnitte 2, 3 stattfindet. Insbesondere eine unerwünschte Gratbildung im Bereich der Mündungsöffnung 4 kann vermieden werden. Auch eine unerwünschte Undichtigkeit im Bereich der Mündungsöffnung 4 kann vermieden werden.

Im Bereich der Mündungsöffnung 4 ergibt sich insbesondere keine unerwünschte Blasenbildung des Materials der Schlauchabschnitte 2, 3.

Vorstehend wurde die biokompatible und/oder lebensmittelechte Schlauch-Baugruppe am Beispiel eines T-Stücks beschrieben. Die biokompatible und/oder lebensmittelechte Schlauch-Baugruppe kann auch als Y-Stück ausgeführt sein. In diesem Fall ist der erste Schlauchabschnitt gebildet durch die beiden oberen Zweige des "Y" und der zweite Schlauchabschnitt durch die untere Spitze des "Y". Eine weitere Ausführung der biokompatiblen und/oder lebensmittelechten Schlauch-Baugruppe kann als X-Stück gestaltet sein. Hier liegen dann vier zu verbindende Schlauch-Anschlüsse vor. Der erste Schlauchabschnitt ist in diesem Falle gebildet durch einen der durchgehenden Abschnitte der Variante "X-Stück". In diesem durchgehenden Schlauchabschnitt mündet dann der zweite, kreuzende Schlauchabschnitt der Variante "X-Stück" über zwei Mündungsöffnungen ein. Beide Mündungsöffnungen können dann über einen entsprechenden Mündungsdichtkörper gemäß dem verfügen, was vorstehend in Zusammenhang mit der Variante "T-Stück" erläutert wurde.

## Patentansprüche

1. Biokompatible und/oder lebensmittelechte Schlauch-Baugruppe (1) zur Verbindung mindestens dreier Schlauch-Anschlüsse (A, B, C),
- mit einem ersten aus Silikonmaterial gefertigten Schlauchabschnitt (2) zur Verbindung von zweien (A, B) der mindestens drei Schlauch-Anschlüsse (A, B, C),
- mit einem zweiten aus Silikonmaterial gefertigten Schlauchabschnitt (3), der über eine Mündungsöffnung (4) in einer Mantelwand (5) des ersten Schlauchabschnitts (2) in diesen einmündet,
- mit einem aus Silikonmaterial gefertigten Mündungsdichtkörper (8), der die Mündungsöffnung (4) zum Innenlumen (6) des ersten Schlauchabschnitts (2) und zum Innenlumen (7) des zweiten Schlauchabschnitts (3) hin begrenzt,
- wobei der erste Schlauchabschnitt (2) und der zweite Schlauchabschnitt (3) an den Mündungsdichtkörper (8) angespritzt sind,
- wobei der Mündungsdichtkörper (8) einen Hülsenabschnitt (8a), der längs des zweiten Schlauchabschnitts (3) verläuft, und einen weiteren Dichtkörperabschnitt (8b) aufweist, der die Mündungsöffnung (4) umgibt und als Teilumfangs-Hülse ausgebildet ist, welche einem Teil eines Umfangs des ersten Schlauchabschnitts (2) folgt.

2. Schlauch-Baugruppe nach Anspruch 1, **dadurch gekennzeichnet, dass** der weitere Dichtkörperabschnitt (8b) die Form eines Halbkreis-Zylindermantels aufweist, der im Bereich der Mündungsöffnung (4) das Innenlumen (6) des ersten Schlauchabschnitts (2) über einen Teil des Innenlumen-Umfangs des Innenlumens (6) begrenzt.

3. Schlauch-Baugruppe nach Anspruch 2, **dadurch gekennzeichnet, dass** der weitere Dichtkörperabschnitt (8b) im Bereich der Mündungsöffnung (4) das Innenlumen (6) des ersten Schlauchabschnitts (2) über eine Hälfte des Innenlumen-Umfangs des Innenlumens (6) begrenzt.

4. Schlauch-Baugruppe nach einem der Ansprüche 1 bis 3, **gekennzeichnet durch** eine der folgenden Ausführungen:
- T-Stück
- Y-Stück
- X-Stück.

5. Schlauch-Baugruppe nach einem der Ansprüche 1 bis 4, **dadurch gekennzeichnet, dass** eine Shore-Härte des Mündungsdichtkörpers (8) größer ist als eine Shore-Härte der Schlauchabschnitte (2, 3).

6. Schlauch-Baugruppe nach Anspruch 5, **dadurch gekennzeichnet, dass** eine Shore-Härte des Mündungsdichtkörpers (8) im Bereich zwischen 50 Shore-A und 80 Shore-A liegt.

7. Verfahren zum Spritzgießen oder Pressen einer biokompatiblen und/oder lebensmittelechten Schlauch-Baugruppe (1) zur Verbindung mindestens dreier Schlauch-Anschlüsse (A, B, C),
- mit einem ersten aus Silikonmaterial gefertigten Schlauchabschnitt (2) zur Verbindung von zweien (A, B) der mindestens drei Schlauch-Anschlüsse (A, B, C),
- mit einem zweiten aus Silikonmaterial gefertigten Schlauchabschnitt (3), der über eine Mündungsöffnung (4) in einer Mantelwand (5) des ersten Schlauchabschnitts (2) in diesen einmündet,
- mit einem aus Silikonmaterial gefertigten Mündungsdichtkörper (8), der die Mündungsöffnung (4) zum Innenlumen (6) des ersten Schlauchabschnitts (2) und zum Innenlumen (7) des zweiten Schlauchabschnitts (3) hin begrenzt,
- wobei der erste Schlauchabschnitt (2) und der zweite Schlauchabschnitt (3) an den Mündungsdichtkörper (8) angespritzt sind, mit folgenden Schritten:
- Bereitstellen einer Spritzguss- oder Press-Matrize (10) zur Vorgabe der Innenlumen (6, 7) der Schlauchabschnitte (2, 3),
- Verbinden des Mündungsdichtkörpers (8) mit der Matrize (10) im Bereich der Mündungsöffnung (4) der medizinischen Schlauch-Baugruppe (1) zur Vervollständigung einer zu umspritzenden Spritzmatrize,
- Einlegen der Matrize (10) mit dem Mündungsdichtkörper (8) in eine Spritzguss- oder Press-Form,
- Umspritzen der Matrize (10) und des Mündungsdichtkörpers (8) mit fließfähigem Material der Schlauchabschnitte (2, 3) in der Form,
- Ziehen der Matrize (10).

8. Verfahren nach Anspruch 7 zum Spritzgießen oder Pressen einer biokompatiblen und/oder lebensmittelechten Schlauch-Baugruppe nach einem der Ansprüche 1 bis 6.

## Claims

1. A biocompatible and/or food-safe tube assembly (1) for connecting at least three tube connectors (A, B, C), comprising:
- a first tube section (2) made of silicone material for connecting two (A, B) of the at least three tube connectors (A, B, C);
- a second tube section (3) made of silicone material which opens into the first tube section (2) via an orifice (4) in a jacket wall (5) of the first tube section (2);
- an orifice sealing body (8) made of silicone material which delimits the orifice (4) to the inner lumen (6) of the first tube section (2) and to the inner lumen (7) of the second tube section (3),
- wherein the first tube section (2) and the second tube section (3) are molded onto the orifice sealing body (8),
- wherein the orifice sealing body (8) comprises a sleeve section (8a) which extends along the second tube section (3) and a further sealing body section (8b) which surrounds the orifice (4) and is designed as a partial peripheral sleeve which extends along a part of a circumference of the first tube section (2).

2. The tube assembly according to claim 1, **characterized in that** the further sealing body section (8b) comprises the shape of a semicircular cylinder jacket which, in a region of the orifice (4), delimits the inner lumen (6) of the first tube section (2) over part of an inner lumen circumference of the inner lumen (6).

3. The tube assembly according to claim 2, **characterized in that** the further sealing body section (8b) in the region of the orifice (4) delimits the inner lumen (6) of the first tube section (2) over half of the inner lumen circumference of the inner lumen (6).

4. The tube assembly according to any of claims 1 to 3, **characterized in that** the tube assembly is
- a T-piece,
- a Y-piece, or
- an X piece.

5. The tube assembly according to any of claims 1 to 4, **characterized in that** a Shore hardness of the orifice sealing body (8) is greater than a Shore hardness of the tube sections (2, 3).

6. The tube assembly according to claim 5, **characterized in that** a Shore hardness of the orifice sealing body (8) is in a range between 50 Shore-A and 80 Shore-A.

7. A method for injection molding or pressing a biocompatible and/or food-safe tube assembly (1) for connecting at least three tube connectors (A, B, C), comprising
- a first tube section (2) made of silicone material for connecting two (A, B) of the at least three tube connectors (A, B, C),
- a second tube section (3) made of silicone material which opens into the first tube section (2) via an orifice (4) in a jacket wall (5) of the first tube section (2), and
- an orifice sealing body (8) made of silicone material which delimits the orifice (4) to the inner lumen (6) of the first tube section (2) and to the inner lumen (7) of the second tube section (3),
- wherein the first tube section (2) and the second tube section (3) are molded onto the orifice sealing body (8),
the method comprising:
- providing an injection molding or pressing core (10) for defining the inner lumen (6) of the tube sections (2, 3),
- connecting the orifice sealing body (8) to the core (10) in an area of the orifice (4) of the tube assembly (1) to complete a core to be overmolded,
- inserting the core (10) with the orifice sealing body (8) in an injection or pressing mold,
- overmolding the core (10) and the orifice sealing body (8) with flowable material of the tube sections (2, 3) in the mold, and
- pulling the core (10).

8. The method of claim 7 for injection molding or pressing a biocompatible and/or food-safe tube assembly according to any of claims 1 to 6.

## Revendications

1. Ensemble tube biocompatible et/ou résistant aux aliments (1), destiné à raccorder au moins trois raccords de tube (A, B, C),
- avec une première section de tube (2) réalisée en silicone et destinée à relier deux (A, B) des au moins trois raccords de tube (A, B, C),
- avec une deuxième section de tube (3) réalisée en silicone, qui débouche dans la première section de tube (2) par l'intermédiaire d'une ouverture d'embouchure (4) pratiquée dans une paroi extérieure (5) de ce dernier,
- avec un corps d'étanchéité d'embouchure (8) réalisé en silicone, qui délimite l'ouverture d'embouchure (4) vers la lumière interne (6) de la première section de tube (2) et vers la lumière interne (7) de la deuxième section de tube (3),
- la première section de tube (2) et la deuxième section de tube (3) étant moulées par injection sur le corps d'étanchéité d'embouchure (8),
- le corps d'étanchéité d'embouchure (8) comportant une partie en forme de manchon (8a), qui s'étend le long de la deuxième section de tube (3), et une autre partie de corps d'étanchéité (8b) qui entoure l'ouverture d'embouchure (4) et est conçue comme un manchon partiellement circonférentiel qui épouse une partie de la circonférence de la première section de tube.

2. L'ensemble tube selon la revendication 1, **caractérisé en ce que** l'autre partie de corps d'étanchéité (8b) a la forme d'une enveloppe cylindrique semi-circulaire qui, au niveau de l'ouverture d'embouchure (4), délimite la lumière interne (6) de la première section de tube (2) sur une partie de la circonférence de cette lumière interne (6).

3. L'ensemble tube selon la revendication 2, **caractérisé en ce que** l'autre partie de corps d'étanchéité (8b) délimite, au niveau de l'ouverture d'embouchure (4), la lumière interne (6) de la première section de tube (2) sur une moitié de la circonférence de cette lumière interne (6).

4. L'ensemble tube selon l'une quelconque des revendications 1 à 3, **caractérisé par** l'une des configurations suivantes :
- en T ;
- en Y ;
- en X.

5. L'ensemble tube selon l'une quelconque des revendications 1 à 4, **caractérisé en ce que** la dureté Shore du corps d'étanchéité d'embouchure (8) est supérieure à celle des sections de tube (2, 3).

6. L'ensemble tube selon la revendication 5, **caractérisé en ce qu'**une dureté Shore du corps d'étanchéité d'embouchure (8) se situe dans une plage entre 50 Shore A et 80 Shore A.

7. Procédé de moulage par injection ou de pressage d'un ensemble tube (1) biocompatible et/ou résistant aux aliments, destiné à raccorder au moins trois raccords de tube (A, B, C),
- avec une première section de tube (2) réalisée en silicone et destinée à relier deux (A, B) des au moins trois raccords de tube (A, B, C),
- avec une deuxième section de tube (3) réalisée en silicone, qui débouche dans la première section de tube (2) par l'intermédiaire d'une ouverture d'embouchure (4) pratiquée dans une paroi extérieure (5) de ce dernier,
- avec un corps d'étanchéité d'embouchure (8) réalisé en silicone, qui délimite l'ouverture d'embouchure (4) vers la lumière interne (6) de la première section de tube (2) et vers la lumière interne (7) de la deuxième section de tube (3),
- la première section de tube (2) et la deuxième section de tube (3) étant moulées par injection sur le corps d'étanchéité d'embouchure (8), comprenant les étapes suivantes :
- mise à disposition d'une matrice de moulage par injection ou de pressage (10) destinée à définir les lumières internes (6, 7) des sections de tube (2, 3),
- raccordement du corps d'étanchéité d'embouchure (8) avec la matrice (10) au niveau de l'ouverture d'embouchure (4) de l'ensemble tube médical (1) afin de compléter une matrice de moulage par injection destinée à être surmoulée,
- mise en place de la matrice (10) avec le corps d'étanchéité d'embouchure (8) dans un moule d'injection ou de pressage,
- surmoulage de la matrice (10) et du corps d'étanchéité d'embouchure (8) avec le matériau fluide des sections de tube (2, 3) dans le moule,
- retrait de la matrice (10).

8. Le procédé selon la revendication 7, destiné au moulage par injection ou au pressage d'un ensemble tube biocompatible et/ou résistant aux aliments selon l'une quelconque des revendications 1 à 6.
